(19) Europäisches Patentamt European Patent Office Office européen des brevets

(11) **EP 2 261 304 B1**

(12) **EUROPEAN PATENT SPECIFICATION**

(45) Date of publication and mention of the grant of the patent:
**09.05.2012 Bulletin 2012/19**

(51) Int Cl.:
***C09K 15/08*** *(2006.01)*    ***A23L 3/3508*** *(2006.01)*
***C09K 15/34*** *(2006.01)*

(21) Application number: **10009488.7**

(22) Date of filing: **17.03.2005**

(54) **Degradation inhibitor**

Abbauinhibitor

Inhibiteur de dégradation

(84) Designated Contracting States:
**AT BE BG CH CY CZ DE DK EE ES FI FR GB GR HU IE IS IT LI LT LU MC NL PL PT RO SE SI SK TR**

(30) Priority: **19.03.2004 JP 2004080224**

(43) Date of publication of application:
**15.12.2010 Bulletin 2010/50**

(62) Document number(s) of the earlier application(s) in accordance with Art. 76 EPC:
**05720993.4 / 1 726 632**

(73) Proprietor: **Mitsubishi Chemical Corporation Tokyo 108-0014 (JP)**

(72) Inventor: **Kido, Kirotsugu Yokohama-shi Kanagawa 2278502 (JP)**

(74) Representative: **Duckworth, Timothy John J A Kemp 14 South Square Gray's Inn London WC1R 5JJ (GB)**

(56) References cited:
**WO-A-00/39066**    **US-A- 5 084 293**
**US-A- 5 795 609**    **US-A- 6 066 327**

**Description**

TECHNICAL FIELD

The present invention relates to an anti-degradation agent, and more particularly, to an anti-degradation agent which is also useful as an agent for improving agent a keeping property of various products themselves such as foods and cosmetics.

BACKGROUND ART

Deterioration of substances is caused by a quality change as well as a reaction between components thereof in association with the quality change. These change and reaction are caused at the same time or in a chain-like or serial manner. By a result of studies until now, these deterioration processes relate to oxidation or photo-deterioration. The oxidation or the photo-deterioration is caused under such circumstances in air, in water, at an interface between air and water, at an interface between water and oil or at an interface between air and oil. The factors for accelerating the oxidative deterioration include enzymes, metals and sensitizers. The photo-deterioration is caused when the substances absorb ultraviolet ray, visible light or near-infrared ray. From these, the deterioration of substances is caused owing to combination of these factors.

The beverage or food and the perfume or cosmetic tend to be usually deteriorated by oxidation of perfume components, pigments or other materials blended therein during the production process or storage thereof. It is important to prevent such an oxidation for the purpose of maintaining a good quality of each of the beverage or food and the perfume or cosmetic. For this reason, in the beverage or food and the perfume or cosmetics, there have been used, for example, natural antioxidants, synthetic antioxidants or preparations obtained by appropriately blending these antioxidants with each other (hereinafter totally referred to merely as "antioxidants").

For example, there are generally known antioxidants made of rosmarinic acid, anti-degradation agents made of carnosol or carnosic acid, antioxidants made of herbs such as rosemary, and antioxidants containing vitamin C or vitamin E (e.g., refer to Patent Documents 1 and 2). In addition, there are also known anti-degradation agents containing rosmarinic acid, carnosol and carnosic acid as rosemary extracts. However, among these rosemary extracts, the rosmarinic acid is water-soluble, whereas the carnosol and carnosic acid are water-insoluble. Therefore, if it is intended to prepare such an anti-degradation agent containing all three kinds of the rosemary extracts, the contents of the respective extracts as well as the balance therebetween in the anti-degradation agent can be controlled only to a limited extent owing to the extraction conditions (in particular, a solvent used). More specifically, the content of rosmarinic acid in the anti-degradation agent is about 2% by weight at most, whereas the total content of the carnosol and carnosic acid is about 1% by weight at most. In addition, these antioxidants tend to be unsatisfactory in stability of an oxidation-inhibiting performance thereof against outside environmental conditions.

In particular, in application fields such as food and perfume or cosmetic, it has been strongly required to provide antioxidants which can exhibit a good effect even when added in a small amount and are free from deterioration even upon heating. However, at present, no antioxidants fully satisfying the above requirements are known in the art until now. Further, since the photo-deterioration and the oxidative deterioration are frequently different in mechanism, etc., from each other, the antioxidants may fail to exhibit a satisfactory effect as a photo-deterioration inhibitor even though they are effective as an oxidation preventing agent. In addition, the antioxidants might sometimes undergo photo-deterioration by themselves and, therefore, may fail to exhibit a fully satisfactory effect as the photo-deterioration inhibitor.

Patent Document 1: Japanese Patent Application Laid-open (KOKAI) No. 2002-363557
Patent Document 2: Japanese Patent Application Laid-open (KOKAI) No. 2003-55686

US 5795609 describes a method for obtaining antioxidant substances from vegetable matter by mixing vegetable matter containing antioxidant substances with a $C_2$-$C_6$ alkylene glycol and subjecting the mixture to a pressure of at least 40 bar.

DISCLOSURE OF THE INVENTION

Problem to be Solved by the Invention

The present invention has been made in view of the above conventional problems. An object of the present invention is to provide an anti-degradation agent which has an excellent anti-degradation performance for food, cosmetic, etc., and can exhibit a good effect even when added in a small amount, show a high heat resistance, and is free from adverse influence due to light.

Means for Solving Problem

As a result of the present inventors' earnest study for solving the above conventional problems, it has been found that the above object can be achieved by such a composition containing a water-soluble antioxidant and a water-insoluble antioxidant which may further contain an emulsifying agent, or contain a specific water-insoluble antioxidant at a high concentration. In addition, it has also been found that these compositions can exhibit common properties defined by predetermined parameters.

The present invention has been attained on the basis of the above findings. Specifically, the present invention provides:

[1] An anti-degradation agent comprising rosmarinic acid, and carnosol and/or carnosic acid, a total content of the carnosol and the carnosic acid being not less than 4% by weight.
[2] An anti-degradation agent according to [1], wherein a content of the rosmarinic acid is not less than 0.5% by weight.
[3] An anti-degradation agent according to [1] or [2], wherein a weight ratio of the rosmarinic acid to a sum of the carnosol and the carnosic acid is 10/1 to 1/99.
[4] A beverage or food comprising the anti-degradation agent as defined in any one of [1] to [3] in an amount of 0.0001 to 30% by weight.
[5] A diet or pet food comprising the anti-degradation agent as defined in any one of [1] to [3] in an amount of 0.0001 to 30% by weight.
[6] A perfume or cosmetic comprising the anti-degradation agent as defined in any one of claims 1 to 3 in an amount of 0.0001 to 30% by weight.
[7] A glaze agent comprising the anti-degradation agent as defined in any one of [1] to [3] in an amount of 0.0001 to 30% by weight.
[8] A plastic product comprising the anti-degradation agent as defined in any one of [1] to [3] in an amount of 0.00001 to 20% by weight.
[9] A process which comprises applying to a beverage or food the anti-degradation agent as defined in any one of [1] to [3].
[10] A process which comprises applying to a diet or pet food the anti-degradation agent as defined in any one of [1] to [3].
[11] A process which comprises applying to a perfume or cosmetic the anti-degradation agent as defined in any one of [1] to [3].
[12] A process which comprises applying to a glaze agent the anti-degradation agent as defined in any one of [1] to [3].
[13] A process which comprises applying to a plastic product the anti-degradation agent as defined in any one of [1] to [3].
[14] A process as defined in any one of [9] to [12] wherein the anti-degradation agent is applied in an amount of 0.0001 to 30% by weight.
[15] A process as defined in [13] wherein the anti-degradation agent is applied in an amount of 0.00001 to 20% by weight.

EFFECT OF THE INVENTION

The anti-degradation agent of the present invention has a high safety, is usable even in a small amount, exhibits a high heat resistance, and is excellent in oxidative deterioration preventing property and photo-deterioration preventing property for foods, cosmetics, etc.

PREFERRED EMBODIMENTS FOR CARRYING OUT THE INVENTION

The present invention is described in detail below. The embodiments explained below are only typical examples of the present invention, and not intended to limit the scope of the present invention.

The anti-degradation agent according to the present invention contains the water-soluble antioxidant rosmarinic acid, and the water-insoluble antioxidant(s) carnosol and/or carnosic acid, and the total content of the carnosol and the carnosic acid therein is not less than 4% by weight.

(Water-insoluble antioxidant)

The water-insoluble antioxidant used in the present invention means an antioxidant having a solubility in 100 g of water of less than 0.1 g, preferably not more than 0.05 g and more preferably not more than 0.01 g as measured at 25°C. Examples of water-insoluble antioxidants include tea extracts, catechin, epicatechin, epigallocatechin, catechin gallate, epigallocatechin gallate, vitamin E ($\alpha$, $\beta$, y, $\delta$-tocopherol), mixed tocopherol and vitamin C fatty esters. Further, carnosol and carnosic acid are present as water-insoluble antioxidant in the agent of the invention. The details of the carnosol and carnosic acid are described hereinlater.

(Water-soluble antioxidant)

The water-soluble antioxidant used in the present invention means an antioxidant having a solubility in 100 g of water of usually not less than 0.1 g, preferably not less than 0.5 g, more preferably not less than 1 g and still more preferably not less than 5 g as measured at 25°C. Examples of water-soluble antioxidants include water-soluble natural extracts such as water-soluble rosemary extracts, and vitamin C.

The rosmarinic acid is one of phenol-carboxylic acids contained in herbs, in particular, contained in a large amount in rosemary. The rosmarinic acid has such a structure in which two phenol-carboxylic acids are bonded to each other. Therefore, the rosmarinic acid structurally and functionally exhibits a higher oxidation-inhibiting effect than those of phenol-carboxylic acids such as ferulic acid, caffeic acid and chlorogenic acid because of a larger number of phenolic hydroxyl groups contained therein. Further, the rosmarinic acid exhibits a high activation effect for inhibition of enzyme reaction like SOD (superoxide dimustase). In addition, the rosmarinic acid also has a high photo-deterioration inhibiting effect because of conjugated double bond contained in a structure thereof.

The rosmarinic acid used in the present invention is preferably in the form of a natural extract and more preferably a glycoside formed by bonding a sugar to the rosmarinic acid from the viewpoint of safety. Thus, the rosmarinic acid used in the present invention also includes the glycosides of rosmarinic acid. The glycosides having any structure may be used in the present invention. Natural products of the rosmarinic acid may be extracts obtained from herbs, in particular, lamiaceous plants and preferably extracts obtained from rosemary containing a large amount of rosmarinic acid.

The general production method of rosmarinic acid is as follows. As the raw material, there may be used a whole grass of rosemary, or any of leaves, roots, stems, flowers, fruits and seeds of rosemary. Among them, preferred are leaves of rosemary. In order to enhance the extraction efficiency, rosemary may be usually used in the form of cut pieces. The rosmarinic acid is obtained as a water-soluble extract of rosemary. Therefore, the rosmarinic acid may be produced by subjecting rosemary to extraction treatment with hexane, hexane/ethanol, ethanol, hydrated ethanol or supercritical carbon dioxide, adding water to the resultant extract to precipitate water-insoluble components therefrom, and concentrating the obtained solution from which the water-insoluble components are removed, under reduced pressure. As the hydrated ethanol, there may be preferably used those having a water content of 40 to 60% by weight.

(Carnosol and carnosic acid)

The carnosol and carnosic acid are contained in a large amount in not only rosemary but also herb-based condiments such as sage, thyme and oregano. The carnosol and carnosic acid have an abietane structure containing an isoprene skeleton unlike the other antioxidants and, therefore, exhibit a considerably high oxidation-inhibiting effect on fats and oils, etc., as compared to the other antioxidants. In addition, the carnosol and carnosic acid have a conjugated double bond in a structure thereof and further a tautomerism structure. Therefore, the carnosol and carnosic acid tend to be structurally stabilized against radicals even when undergoing influence of the radicals and, as a result, can exhibit a high photo-deterioration inhibiting effect.

The carnosol and carnosic acid used in the present invention are preferably in the form of a natural extract from the viewpoint of safety. Natural products of the carnosol and carnosic acid may be extracts obtained from herb-based plants such as sage, thyme and oregano and are preferably extracts obtained from rosemary containing a large amount of carnosol and carnosic acid.

The carnosol and carnosic acid may be obtained as a water-insoluble extract of rosemary. An example of the general production method of the carnosol and carnosic acid is as follows. First, similarly to the above water-soluble extracts, rosemary is subjected to extraction treatment with hexane, hexane/ethanol, ethanol, hydrated ethanol or supercritical carbon dioxide, and then water is added to the resultant extract to precipitate water-insoluble components therefrom. After the resultant mixture is mixed with activated carbon and then stirred, a mixture of the water-insoluble components and activated carbon is separated from the extract. The resultant mixture is further subjected to extract treatment with hexane, hexane/ethanol, ethanol, hydrated ethanol or supercritical carbon dioxide, and the obtained extract was distilled to remove the extraction solvent therefrom, thereby obtaining the carnosol and carnosic acid in the form of a powdered concentrate. The details of the above method for production of the carnosol and carnosic acid will become more apparent by referring to descriptions of Japanese Patent Publication (KOKOKU) No. 59-4469.

(Emulsifying agent)

As an emulsifying agent, there may be used any of conventional emulsifying agents which have been used in application fields of foods, diets, cosmetics, drugs or medicines, industrial products, etc. That is, the emulsifying agent means an amphiphatic compound containing both a hydrophilic group moiety and a hydrophobic group moiety therein which are chemically bonded to each other. The hydrophilic group moiety may be either ionic, nonionic or amphoteric, and further may be a hydrophilic polymer. The hydrophobic group moiety may be constituted from typically a fatty acid having usually 2 to 40 carbon atoms and preferably 6 to 24 carbon atoms. Also, the hydrophobic group moiety may be constituted from not fatty acid but cholesterol group or hydrophobic polymer.

Examples of the emulsifying agents for foods may include sucrose fatty esters, glycerin fatty esters, polyglycerin fatty esters, organic acid glycerin fatty esters, lactic acid fatty esters and sorbitan fatty esters. Examples of the emulsifying agents produced from natural products may include lecithins such as vegetable lecithin, yolk lecithin, fractional lecithin and enzyme-treated lecithin; saponins such as saponin, Quilaja saponin and soybean saponin; phospholipids such as sphingolipids, vegetable sterols and animal sterols; bile powder; and glycolipids such as tomato glycolipid.

The lower limit of a total content of the carnosol and carnosic acid in the anti-degradation agent is not less than 4% by weight and becomes more preferred in the order of 5% by weight, 6% by weight, 8% by weight, 12% by weight and 20% by weight. On the other hand, the upper limit of the total content of the carnosol and carnosic acid in the anti-degradation agent is usually 95% by weight and preferably 80% by weight.

In the anti-degradation agent, the weight ratio of the content of the water-soluble antioxidant to the total content of the carnosol and carnosic acid is usually 10/1 to 1/99 and preferably 1/2 to 1/30. When the weight ratio is controlled to the above-specified range, the resultant anti-degradation agent can be prevented from being deteriorated in degradation-inhibiting performance thereof owing to external environments, and can also be enhanced in stability to light and heat. When the weight ratio of the water-soluble antioxidant is too small, the obtained anti-degradation agent tends to be deteriorated in oxidation-inhibiting performance at an interface between water and oil. On the other hand, when the weight ratio of the water-soluble antioxidant is too large, the obtained anti-degradation agent tends to be lowered in degradation-inhibiting performance for oils and fats.

(Other components)

The anti-degradation agent of the present invention may also contain other optional components, for example, those components mixed therein owing to the methods used for procuring rosmarinic acid, carnosol and carnosic acid. More specifically, the anti-degradation agent may contain optional components which may be extracted upon subjecting herbs to extraction treatment. Further, in addition to the above components, the anti-degradation agent may be used in combination with emulsifying agents, e.g., polyglycerin fatty esters such as polyglycerin lauric esters, polyglycerin myristic esters, polyglycerin palmitic esters, polyglycerin stearic esters and polyglycerin oleic esters, and sucrose fatty esters such as sucrose lauric esters, sucrose myristic esters, sucrose palmitic esters, sucrose stearic esters and sucrose oleic esters; and natural emulsifying agents such as lecithin, phospholipid, cholesterol and licorice. The content of these components is usually 1 to 40% by weight, preferably 3 to 30% by weight and more preferably 5 to 20% by weight.

Further, the anti-degradation agent of the present invention may be used in combination with the other antioxidants such as coffee bean extracts, sunflower extracts, grape seeds, $\alpha$G rutin, catechin and green tea extracts. These other antioxidants may further contain vitamin C, vitamin E (tocopherol), vitamin P and chlorogenic acid. The content of the other antioxidants is usually 0.1 to 50% by weight, preferably 0.5 to 30% by weight and more preferably 1 to 20% by weight. Further, the anti-degradation agent of the present invention may be used in combination with sugar alcohols such as "Oligotose", trehalose, xylitol and erythritol, and sugars. The content of the sugar alcohols or sugars is usually 0.1 to 50% by weight, preferably 0.5 to 30% by weight and more preferably 1 to 20% by weight.

The method for producing the anti-degradation agent of the present invention is not particularly limited, but is preferably the above extraction method in which herbs such as rosemary are used as the raw material. Also, there may be used the method of mixing different extracts obtained by varying extraction conditions with each other.

The anti-degradation agent of the present invention may be usually used in the form of a solution obtained by dissolving the above respective components in water or a mixed solvent of water and ethanol. When using carnosol and carnosic acid in combination, both the compounds are usually dissolved in the mixed solvent of water and ethanol. The solution of the carnosol and carnosic acid in the mixed solvent may be usually prepared by mixing the above respective components with each other, adding ethanol to the resultant mixture, and then adding water to the ethanol solution. The mixing ratio of water to ethanol in the mixed solvent is usually 1:1 to 3:1. The anti-degradation agent may be in the form of a powder. Such a powder of the anti-degradation agent may be produced by spray-drying or freeze-drying the above solution.

The anti-degradation agent of the present invention may be suitably applied to foods and cosmetics which tend to be readily deteriorated in quality. In this case, the amount of the anti-degradation agent added is usually 0.0001 to 30% by weight, preferably 0.0003 to 10% by weight and more preferably 0.0005 to 5% by weight on the basis of the weight of the respective products.

<u><Method for evaluating performance of anti-degradation agent of the present invention></u>

The photo resistance and the heat resistance of the anti-degradation agents can be evaluated according to the formulae (1) and (2) or the formulae (3) and (4).

The anti-degradation agent preferably exhibits a photo resistance of not less than 10 and a heat resistance of not less than 10. Both of the photo resistance and the heat resistance of the anti-degradation agent are more preferably not less than 20.

The formulae (1) to (4) are set out in definitions (A) to (D) below:

(A) When a food as a sample is placed in a container and irradiated with light at a total illuminance of 500,000 lux to measure an amount of hexanal generated from the food and accumulated within a head space of the container by gas chromatography, said photo resistance being calculated from the following formula (1):

$$\text{Photo resistance} = [\{(\text{Amount of hexanal generated from the light-irradiated food}$$
$$\text{containing no anti-degradation agent}) - (\text{Amount of hexanal generated from the}$$
$$\text{light-irradiated food containing the anti-degradation agent})\}/\{(\text{Amount of hexanal}$$
$$\text{generated from the light-irradiated food containing no anti-degradation agent}) -$$
$$(\text{Amount of hexanal generated from the light-non-irradiated food containing the}$$
$$\text{anti-degradation agent})\}] \times 100 \cdots (1).$$

(B) When a food as a sample is placed in a container, air is blown into the container, and the food is maintained at a temperature of 60°C to measure a time at which an amount of volatile components generated from the food is rapidly increased (degradation induction time) using an electrical conductivity meter, said heat resistance being calculated from the following formula (2):

$$\text{Heat Resistance} = [\{(\text{Degradation induction time of the food containing the}$$
$$\text{anti-degradation agent upon heating}) - (\text{Degradation induction time of the food}$$
$$\text{containing no anti-degradation agent upon heating})\}/(\text{Degradation induction time of}$$
$$\text{the food containing no anti-degradation agent upon heating})] \times 100 \cdots (2).$$

The photo resistance and the heat resistance are evaluation parameters adopted on the basis of the following facts. That is, among ordinary deterioration phenomena, in those due to thermal degradation, rapid generation of volatile components tends to be caused when exceeding a certain threshold value of time, resulting in occurrence of rapid deterioration. For this reason, in the present invention, the threshold value of time is utilized as a degradation induction time. In the photo-deterioration, oils and fats or proteins used in foods tend to undergo deterioration when irradiated with light, so that fatty acids or amino acids contained therein are decomposed to produce an aldehyde. At this time, the amount of hexanal which has a low threshold value of time and is contained in a large amount in the deteriorated foods, is measured to determine the change thereof between before and after the light irradiation, and is utilized to evaluate a photo-deterioration preventing property, i.e., a photo resistance.

(C) When a solution of a pigment as a sample filled in a container is irradiated with light at a total illuminance of 500,000 lux to determine a degree of discoloration of the pigment by measuring an absorbance of the pigment solution at a specific wavelength, said photo resistance being calculated from the following formula (3):

Photo resistance = [ {(Absorbance of the light-irradiated pigment solution containing the anti-degradation agent) - (Absorbance of the light-irradiated pigment solution containing no anti-degradation agent)}/{(Absorbance of the light-non-irradiated pigment solution containing the anti-degradation agent) - (Absorbance of the light-irradiated pigment solution containing no anti-degradation agent)}] x 100 ⋯ (3).

(D) When a pigment as a sample placed in a container is held at 55°C for one week to determine a degree of discoloration of the pigment by measuring an absorbance of a solution of the pigment at a specific wavelength, said heat resistance being calculated from the following formula (4):

Heat Resistance = [ {(Absorbance of the heated pigment solution containing the anti-degradation agent) - (Absorbance of the heated pigment solution containing no anti-degradation agent)}/{(Absorbance of the non-heated pigment solution containing the anti-degradation agent) - (Absorbance of the heated pigment solution containing no anti-degradation agent)}] x 100 ⋯ (4).

As foods serving for measuring the photo resistance and heat resistance of the anti-degradation agent, there may be usually used a mixture of 30 parts by weight of soybean oil, 50 parts by weight of wheat flour and 20 parts by weight of water. Upon the respective measurements, there may be used two kinds of test specimens, i.e., one specimen prepared by mixing the above mixture with 0.1 part by weight of the anti-degradation agent and mechanically kneading these components with each other, and the other specimen prepared by mechanically kneading the above mixture solely without adding no anti-degradation agent thereto. Also, in the above definitions, the light with a total illuminance of 500,000 lux may be achieved, for example, by irradiating light with an illuminance of 20,000 lux for 25 hr. The time at which the amount of volatile components is rapidly increased (degradation induction time) may be determined by detecting generation of volatile components using an electrical conductivity meter (e.g., "RANCIMAT 743 MODEL" manufactured by Metronome Inc.).

Products using the anti-degradation agent of the present invention

(Beverage or food)

As the beverage or food of the present invention, there may be suitably used foods which tend to be readily deteriorated in quality. Specific examples of the beverage or food may include beverages, milk beverages, alcohol beverages, boiled rice, beans (such as rice, wheat, barley, corn, millet and barnyard millet), bread and other wheat flour products, noodles, roux such as curry roux and stew roux, frozen foods, chilled foods, retort foods, dairy products such as ice cream, milk-processed foods, beverages such as milk, soft drinks, carbonated beverages, green teas, black teas, oolong teas, coffees, cocoas, refined sake, beers, sparkling wines, synthetic refined sake, sweet sake (mirin), wines, shoutu, whiskies and vegetable juices, condiments such as miso, soy sauce, vinegar, taste seasoning, dressing, sauce and mayonnaise, processed marine products such as fish paste products, fish ham and sausage, dried bonito and foods boiled down in soy, frozen foods such as frozen boiled rice, frozen noodles, frozen croquette, frozen hamburger, frozen shao-mai, frozen gyoza and frozen gratin, instant foods such as instant noodles, instant soups, instant curry, instant miso soups and instant coffees, and confectioneries such as Japanese-style cakes, Japanese unbaked cakes, Japanese semi-baked cakes, baked cakes, unbaked cakes, semi-baked cakes, candies, chocolates, chewing gums, biscuits, rice cakes, snack cakes, oil cakes and other cakes.

Among the above beverages and foods, the processed marine, livestock or oil and fat products are preferably those products which tend to be readily deteriorated in quality or should be preserved for a long period of time. Specific examples of the processed marine, livestock or oil and fat products may include fresh fishes, dried fishes, overnight-dried fishes, dried mirin-seasoned fishes, pigment-keeping agents for shells, red-meat fishes and Crustacea, minced or ground fish meat, marine paste products, foods of delicate flavor, fish sausage, salt-preserved products, laver, seaweed products, unsaturated polyvalent fatty acids such as $\alpha$-linolenic acid, docosahexaenoic acid (DHA) and eicosapentaenoic acid (EPA) and triglycerides thereof as well as foods, chicken, pork, beef, mutton, sausage, ham and their processed products containing these compounds, corn flakes, instant Chinese noodles, oil cakes using oils and fats, fast spreads, and margarine.

The amount of the anti-degradation agent used in the beverage or food is 0.0001 to 30% by weight, preferably 0.0003 to 10% by weight and more preferably 0.0005 to 5% by weight on the basis of the weight of the beverage or food.

The anti-degradation agent for the beverage or food is contains carnosol and/or carnosic acid. The amount of the carnosol and/or carnosic acid added is usually not less than 0.5 ppm, preferably not less than 5 ppm, more preferably not less than 40 ppm and especially preferably not less than 100 ppm on the basis of the amount of the beverage or food. The upper limit of the amount of the carnosol and/or carnosic acid added is usually 10,000 ppm.

Further, in the anti-degradation agent for the beverage or food, rosmarinic acid is used in combination with the carnosol and/or carnosic acid. The amount of the rosmarinic acid added to the beverage or food is usually not less than 5 ppm, preferably not less than 50 ppm, more preferably not less than 500 ppm and especially preferably not less than 1,000 ppm on the basis of the amount of the beverage or food. The upper limit of the amount of the rosmarinic acid added is usually 100,000 ppm.

(Diet and pet food)

The anti-degradation agent of the present invention may also be applied to diets for livestock and cultured fishes as well as pet foods.

The amount of the anti-degradation agent used in the diet or pet food is 0.0001 to 30% by weight, preferably 0.0003 to 30% by weight and more preferably 0.0005 to 30% by weight on the basis of the weight of the diet or pet food.

The anti-degradation agent for the diet or pet food contains carnosol and/or carnosic acid. The amount of the carnosol and/or carnosic acid added is usually not less than 0.5 ppm, preferably not less than 5 ppm, more preferably not less than 40 ppm and especially preferably not less than 100 ppm on the basis of the amount of the diet or pet food. The upper limit of the amount of the carnosol and/or carnosic acid added is usually 100,000 ppm.

Further, in the anti-degradation agent for the diet or pet food, rosmarinic acid is preferably used in combination with the carnosol and/or carnosic acid. The amount of the rosmarinic acid added is usually not less than 5 ppm, preferably not less than 50 ppm, more preferably not less than 500 ppm and especially preferably not less than 1,000 ppm on the basis of the amount of the diet or pet food. The upper limit of the amount of the rosmarinic acid added is usually 100,000 ppm.

(Perfumes or cosmetic)

As the perfume or cosmetic usable in the present invention, there may be suitably used those perfumes and cosmetics which tend to be readily deteriorated in quality. Specific examples of the perfumes and cosmetics may include humectants, beauty white agents, cleansing solutions, lotions, detergents, softening agents, finishing agents, dish-washing agents, detergents for vegetables and fruits and rinsing agents. The amount of the anti-degradation agent used is 0.0001 to 30% by weight, preferably 0.0003 to 10% by weight and more preferably 0.0005 to 5% by weight on the basis of the weight of the perfume or cosmetic.

(Glaze agent)

The term "glaze" means to coat the surface of landed fish with ice upon freezing the landed fish, and the glaze agent is used for forming a uniform ice coat on the surface of the landed fish. Examples of the glaze agent may include functional water, electrolytic water and UV-treated water. The amount of the anti-degradation agent used in the glaze agent is 0.0001 to 30% by weight, preferably 0.0003 to 10% by weight and more preferably 0.0005 to 5% by weight on the basis of the weight of the glaze agent.

(Plastic product)

The anti-degradation agent of the present invention can be added to a plastic product to indirectly prevent beverage or food, perfume or cosmetic and other products enclosed therein from being deteriorated in quality. Specific examples of the plastic product may include plastic containers for the beverage or food and the perfume or cosmetic, packaging materials for foods such as baran (aspidistra) partitions and packs for preservation of cooked foods, packaging materials for sanitary goods such as deodorants and liquid detergents, white home appliances such as refrigerators, air conditioners, air cleaners and laundry/drying machines, and air conditioning equipments for ships, automobiles, trains, airplanes and buildings. The amount of the anti-degradation agent used in the plastic product is 0.00001 to 20% by weight, preferably 0.0001 to 10% by weight and more preferably 0.0005 to 5% by weight on the basis of the weight of the plastic product.

EXAMPLES

The present invention is described in more detail by Examples. However, it should be noted that the following Examples are only illustrative and not intended to limit the scope of the present invention.

Production Example 1:

10 L of 50% hydrated ethanol was added to 1 kg of rosemary, and the resultant mixture was refluxed under heating for 3 hr and then filtered under a hot condition, thereby obtaining a filtrate. The obtained residue was extracted with 6 L of 50% hydrated ethanol, and this extraction procedure was repeated two times, thereby obtaining filtrates. The obtained filtrates were combined together and mixed with 5 L of water to form a precipitate. The resultant solution was mixed with 100 g of activated carbon, stirred for 1 hr, allowed to stand in a cold place overnight, and then filtered to obtain a filtrate. The thus obtained filtrate was concentrated under reduced pressure, thereby obtaining 120 g of a (water-soluble) rosemary extract (1). As a result, it was confirmed that the rosemary extract (1) had a rosmarinic acid content of 31.6% by weight.

Production Example 2:

10 L of 50% hydrated ethanol was added to 1 kg of rosemary, and the resultant mixture was refluxed under heating for 3 hr and then filtered under a hot condition, thereby obtaining a filtrate. The obtained residue was extracted with 6 L of 50% hydrated ethanol, and this extraction procedure was repeated two times, thereby obtaining filtrates. The obtained filtrates were combined together and mixed with 5 L of water to form a precipitate. The resultant filtrate was mixed with 100 g of activated carbon, stirred for 1 hr, allowed to stand in a cold place overnight, and then filtered to obtain a mixture of a precipitate and activated carbon. The thus obtained mixture was mixed with 4 L of ethanol, refluxed under heating for 3 hr, and then filtered under a hot condition, thereby obtaining a filtrate. The obtained residue was extracted with 2.4 L of ethanol, and this extraction procedure was repeated two times, thereby obtaining filtrates. The obtained filtrates were combined together and concentrated under reduced pressure to distill off ethanol therefrom, thereby obtaining a (water-insoluble) powdery rosemary extract (2). As a result, it was confirmed that the rosemary extract (2) had a total content of carnosol and carnosic acid of 24.9% by weight.

Production Example 3:

10 L of ethanol was added to 1 kg of rosemary, and the resultant mixture was refluxed under heating for 3 hr and then filtered under a hot condition, thereby obtaining a filtrate. The obtained residue was extracted with 6 L of ethanol, and this extraction procedure was repeated two times, thereby obtaining filtrates. The obtained filtrates were combined together and mixed with 100 g of activated carbon, stirred for 1 hr, allowed to stand in a cold place overnight, and then filtered, thereby obtaining a rosemary extract (3). As a result, it was confirmed that the rosemary extract (3) had a rosmarinic acid content of 0.25% by weight and a total content of carnosol and carnosic acid of 2.9% by weight.

Examples 1 to 3 and Comparative Examples 1 to 3:

<Production of kneaded material>

A kneaded material composed of 30 parts by weight of a soybean oil, 50 parts by weight of a wheat flour and 20 part by weight of water was subjected to deterioration test. More specifically, the respective anti-degradation agent samples shown in Table 2 were added to a mixture composed of 30 parts by weight of a soybean oil, 50 parts by weight of a wheat flour and 20 part by weight of water in a total amount of 0.1% by weight based on the weight of the mixture (at a mixing ratio shown in Table 2), and the resultant mixture was kneaded together by hands until a uniform kneaded material was produced. The heat resistance (oxidation odor-preventing property) of the thus produced kneaded material was evaluated according to the above formula (2). The results are shown in Table 2. During the evaluation test, the kneaded material was visually observed to determine a change in color (brown discoloration-preventing property) thereof.

The evaluation criteria are shown in Table 1 below. The results are shown in Table 2.

Table 1

| Color of kneaded material | Brown discoloration-preventing property |
|---|---|
| Similar to color upon no addition | 0 |
| Slightly lighter than color upon no addition | 20 |
| Apparently lighter than color upon no addition | 50 |
| Slightly tinted | 80 |
| Not tinted | 100 |

Table 2

| | Examples | | | Comparative Examples | | |
|---|---|---|---|---|---|---|
| | 1 | 2 | | 3 1 | 2 | 3 |
| Rosemary extract (1) (wt%) | 80 | 50 | 5 | 0 | 100 | 0 |
| Rosemary extract (2) (wt%) | 20 | 50 | 95 | 0 | 0 | 100 |
| Rosemary extract (3) (wt%) | 0 | 0 | 0 | 100 | 0 | 0 |
| rosmarinic acid (A) (wt%) | 25.3 | 15.8 | 1.6 | 0.25 | 31.6 | 0 |
| Carnosol + carnosic acid {(B) + (C)} (wt%) | 4.98 | 12.5 | 24 | 2.9 | 0 | 24.9 |
| {(B) + (C)}/(A) | 0.2 | 0.79 | 15 | 11.6 | (100/0) | 0 |
| Evaluation | | | | | | |
| Oxidation odor-preventing property | 10 | 18 | 20 | -1 | 4 | 22 |
| Brown discoloration-preventing property | 100 | 100 | 80 | 50 | 100 | 50 |
| Total evaluation | B | B | A | C | C | C |

In Table 2, the "total evaluation" was conducted using ratings shown in Table 3 below.

Table 3

| A | Oxidation odor-preventing property of not less than 20 and a brown discoloration-preventing property of not less than 80 (highly effective) |
|---|---|
| B | Oxidation odor-preventing property of not less than 10 and a brown discoloration-preventing property of not less than 80 (effective) |
| C | Other than the above cases (ineffective) |

From the above results, it has been apparently recognized that the anti-degradation agents of the present invention were able to prevent generation of oxidation odor due to deterioration as well as brown discoloration due to heat.

Example 4:

A kneaded material composed of 30 parts by weight of a soybean oil, 50 parts by weight of a wheat flour and 20 part by weight of water was subjected to deterioration test. More specifically, the respective anti-degradation agent samples shown in Table 4 were added to a mixture composed of 30 parts by weight of a soybean oil, 50 parts by weight of a wheat flour and 20 part by weight of water in a total amount of 0.1% by weight based on the weight of the mixture, and the resultant mixture was mechanically kneaded together to obtain a kneaded material. The photo resistance and the heat resistance of the thus obtained kneaded material was evaluated according to the above formulae (1) and (2), respectively. The results are shown in Table 4.

Table 4

| Anti-degradation agent sample | Photo resistance | Heat resistance |
|---|---|---|
| Mixture of 80 parts by weight of rosemary extract (1) and 20 parts by weight of rosemary extract (2) | 80 | 12 |
| Mixture of 50 parts by weight of rosemary extract (1) and 50 parts by weight of rosemary extract (2) | 75 | 18 |
| Mixture of 5 parts by weight of rosemary extract (1) and 95 parts by weight of carnosol | 90 | 20 |
| Rosemary extract (3)* | 2 | -1 |
| Rosemary extract (1)* | 4 | 4 |
| Rosemary extract (2)* | 8 | 22 |
| No addition* | 0 | 0 |
| *: For comparison only; not part of the present invention | | |

Example 5:

A red radish pigment was subjected to deterioration test. More specifically, 0.1% of the red radish pigment was added to a 7% alcohol (ethanol) aqueous solution, and the respective anti-degradation agent samples shown in Table 5 were further added to the solution in a total amount of 0.1% by weight. The thus obtained sample solution was irradiated with light at a total illuminance of 500,000 lux (i.e., light irradiation: 20000 lux x 25 hr) (at 5°C) to evaluate a photo resistance thereof, and allowed to stand at 55°C for one week to evaluate a heat resistance thereof. The degree of discoloration of the pigment was evaluated by measuring an absorbance thereof at a specific wavelength. The photo resistance and the heat resistance were evaluated according to the above formulae (3) and (4), respectively, and expressed by the relative values obtained when each of the photo resistance and heat resistance before the test was regarded as 100. The results are shown in Table 5

Table 5

| Anti-degradation agent sample | Photo resistance | Heat resistance |
|---|---|---|
| Rosemary extract (1)* | 2 | 5 |
| Mixture of 67 parts by weight of rosemary extract (1) and 33 parts by weight of rosemary extract (2) | 23 | 17 |
| Mixture of 40 parts by weight of rosemary extract (1), 20 parts by weight of rosemary extract (2) and 40 parts by weight of polyglycerin monostearic ester | 29 | 19 |
| * For comparison only; not part of present invention | | |

**Claims**

1. An anti-degradation agent comprising rosmarinic acid, and carnosol and/or carnosic acid, a total content of the carnosol and the carnosic acid being not less than 4% by weight.

2. An anti-degradation agent according to claim 1, wherein a content of the rosmarinic acid is not less than 0.5% by weight.

3. An anti-degradation agent according to claim 1 or 2, wherein a weight ratio of the rosmarinic acid to a sum of the carnosol and the carnosic acid is 10/1 to 1/99.

4. A beverage or food comprising the anti-degradation agent as defined in any one of claims 1 to 3 in an amount of 0.0001 to 30% by weight.

5. A diet or pet food comprising the anti-degradation agent as defined in any one of claims 1 to 3 in an amount of 0.0001 to 30% by weight.

6. A perfume or cosmetic comprising the anti-degradation agent as defined in any one of claims 1 to 3 in an amount of 0.0001 to 30% by weight.

7. A glaze agent comprising the anti-degradation agent as defined in any one of claims 1 to 3 in an amount of 0.0001 to 30% by weight.

8. A plastic product comprising the anti-degradation agent as defined in any one of claims 1 to 3 in an amount of 0.00001 to 20% by weight.

9. A process which comprises applying to a beverage or food the anti-degradation agent as defined in any one of claims 1 to 3.

10. A process which comprises applying to a diet or pet food the anti-degradation agent as defined in any one of claims 1 to 3.

11. A process which comprises applying to a perfume or cosmetic the anti-degradation agent as defined in any one of claims 1 to 3.

12. A process which comprises applying to a glaze agent the anti-degradation agent as defined in any one of claims 1 to 3.

13. A process which comprises applying to a plastic product the anti-degradation agent as defined in any one of claims 1 to 3.

14. A process as defined in any one of claims 9 to 12 wherein the anti-degradation agent is applied in an amount of 0.0001 to 30% by weight.

15. A process as defined in claim 13 wherein the anti-degradation agent is applied in an amount of 0.00001 to 20% by weight.


**Patentansprüche**

1. Anti-Abbau-Mittel, das Rosmarinsäure und Carnosol und/oder Carnosolsäure umfasst, wobei der Gesamtgehalt des Carnosol und der Carnosolsäure nicht weniger als 4 Gew.-% ist.

2. Anti-Abbau-Mittel gemäß Anspruch 1, wobei der Gehalt der Rosmarinsäure nicht weniger als 0,5 Gew.-% ist.

3. Anti-Abbau-Mittel gemäß Anspruch 1 oder 2, wobei das Gewichtsverhältnis der Rosmarinsäure zu der Summe des Carnosol und der Carnosolsäure 10/1 bis 1/99 ist.

4. Getränk oder Nahrungsmittel, das das Anti-Abbau-Mittel, wie es in einem der Ansprüche 1 bis 3 definiert ist, in einer Menge von 0,0001 bis 30 Gew.-% umfasst.

5. Diät- oder Tiernahrungsmittel, das das Anti-Abbau-Mittel, wie es in einem der Ansprüche 1 bis 3 definiert ist, in einer Menge von 0,0001 bis 30 Gew.-% umfasst.

6. Parfum oder Kosmetikum, das das Anti-Abbau-Mittel, wie es in einem der Ansprüche 1 bis 3 definiert ist, in einer Menge von 0,0001 bis 30 Gew.-% umfasst.

7. Glasur, die das Anti-Abbau-Mittel, wie es in einem der Ansprüche 1 bis 3 definiert ist, in einer Menge von 0,0001 bis 30 Gew.-% umfasst.

8. Kunststoffprodukt, das das Anti-Abbau-Mittel, wie es in einem der Ansprüche 1 bis 3 definiert ist, in einer Menge von 0,00001 bis 20 Gew.-% umfasst.

9. Verfahren, das Anwenden des Anti-Abbau-Mittels, wie es in einem der Ansprüche 1 bis 3 definiert ist, auf ein Getränk oder ein Nahrungsmittel umfasst.

10. Verfahren, das Anwenden des Anti-Abbau-Mittels, wie es in einem der Ansprüche 1 bis 3 definiert ist, auf ein Diät- oder Tiernahrungsmittel umfasst.

11. Verfahren, das Anwenden des Anti-Abbau-Mittels, wie es in einem der Ansprüche 1 bis 3 definiert ist, auf ein Parfum oder Kosmetikum umfasst.

12. Verfahren, das Anwenden des Anti-Abbau-Mittels, wie es in einem der Ansprüche 1 bis 3 definiert ist, auf eine Glasur umfasst.

13. Verfahren, das Anwenden des Anti-Abbau-Mittels, wie es in einem der Ansprüche 1 bis 3 definiert ist, auf ein Kunststoffprodukt umfasst.

14. Verfahren, wie es in einem der Ansprüche 9 bis 12 definiert ist, wobei das Anti-Abbau-Mittel in einer Menge von 0,0001 bis 30 Gew.-% angewendet wird.

15. Verfahren, wie es in Anspruch 13 definiert ist, wobei das Anti-Abbau-Mittel in einer Menge von 0,00001 bis 20 Gew.-% angewendet wird.


## Revendications

1. Inhibiteur de dégradation qui comprend de l'acide romarinique ainsi que du carnosol et/ou de l'acide carnosique, et dont la teneur totale en carnosol et acide carnosique vaut au moins 4 % en poids.

2. Inhibiteur de dégradation conforme à la revendication 1, dans lequel l'acide romarinique se trouve présent en une proportion d'au moins 0,5 % en poids.

3. Inhibiteur de dégradation conforme à la revendication 1 ou 2, dans lequel le rapport du poids d'acide romarinique à la somme des poids de carnosol et d'acide carnosique vaut de 10/1 à 1/99.

4. Boisson ou aliment comprenant un inhibiteur de dégradation conforme à l'une des revendications 1 à 3, en une proportion de 0,0001 à 30 % en poids.

5. Aliment de régime ou nourriture pour animaux familiers, comprenant un inhibiteur de dégradation conforme à l'une des revendications 1 à 3, en une proportion de 0,0001 à 30 % en poids.

6. Parfum ou produit cosmétique comprenant un inhibiteur de dégradation conforme à l'une des revendications 1 à 3, en une proportion de 0,0001 à 30 % en poids.

7. Agent de glaçage comprenant un inhibiteur de dégradation conforme à l'une des revendications 1 à 3, en une proportion de 0,0001 à 30 % en poids.

8. Produit en plastique comprenant un inhibiteur de dégradation conforme à l'une des revendications 1 à 3, en une proportion de 0,00001 à 20 % en poids.

9. Procédé qui comporte le fait d'introduire dans une boisson ou un aliment un inhibiteur de dégradation conforme à l'une des revendications 1 à 3.

10. Procédé qui comporte le fait d'introduire dans un aliment de régime ou de la nourriture pour animaux familiers un inhibiteur de dégradation conforme à l'une des revendications 1 à 3.

11. Procédé qui comporte le fait d'introduire dans un parfum ou un produit cosmétique un inhibiteur de dégradation conforme à l'une des revendications 1 à 3.

12. Procédé qui comporte le fait d'introduire dans un agent de glaçage un inhibiteur de dégradation conforme à l'une des revendications 1 à 3.

13. Procédé qui comporte le fait d'introduire dans un produit en plastique un inhibiteur de dégradation conforme à l'une des revendications 1 à 3.

14. Procédé conforme à l'une des revendications 9 à 12, dans lequel l'inhibiteur de dégradation est introduit en une proportion de 0,0001 à 30 % en poids.

15. Procédé conforme à la revendication 13, dans lequel l'inhibiteur de dégradation est introduit en une proportion de 0,00001 à 20 % en poids.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002363557 A
- JP 2003055686 A
- US 5795609 A
- JP 59004469 A